# EUROPEAN PATENT SPECIFICATION

(11) **EP 3 614 966 B1**
(45) Date of publication and mention of the grant of the patent: **25.10.2023**
(21) Application number: 18723756.5
(22) Date of filing: 28.04.2018
(51) Int. Cl.: A61F 2/24

(54) **ANNULOPLASTY IMPLANT**
ANNULOPLASTIE-IMPLANTAT
IMPLANT D'ANNULOPLASTIE

(30) Priority: 28.04.2017 US 201762491727 P; 28.04.2017 EP 17168875
(43) Date of publication of application: 04.03.2020
(73) Proprietor: HVR Cardio Oy, 02600 Espoo (FI)
(72) Inventor: ZERKOWSKI, Hans-Reinhard, 8280 Kreuzlingen (CH); KERÄNEN, Olli, 23741 Bjärred (SE); O'CARROLL, Ger, Co. Sligo (IE); PUGH, Mark, Coolaney Co. Sligo (IE); O'REGAN, Jake, Rathrippin Collooney Co. Sligo (IE); DEANE, Stuart, Rathrippin Collooney Co. Sligo (IE)
(74) Representative: Invent Horizon IP
(86) International application number: PCT/EP2018/060996
(87) International publication number: WO 2018/197721

(56) References cited:
- EP-A1- 2 415 421
- WO-A1-03/020178
- WO-A1-2007/030063
- US-A1- 2017 007 402

## Description

### Technical field

This invention pertains in general to the field of cardiac valve replacement and repair. More particularly the invention relates to an annuloplasty implant, such as an annuloplasty ring or helix, for positioning at the heart valve annulus.

### Background

Diseased mitral and tricuspid valves frequently need replacement or repair. The mitral and tricuspid valve leaflets or supporting chordae may degenerate and weaken or the annulus may dilate leading to valve leak. Mitral and tricuspid valve replacement and repair are frequently performed with aid of an annuloplasty ring, used to reduce the diameter of the annulus, or modify the geometry of the annulus in any other way, or aid as a generally supporting structure during the valve replacement or repair procedure.

A problem with prior art annuloplasty implants is to achieve a reliable fixation at the annulus while at the same time accommodating with the movements of the beating heart. I.e. while it could be possible to provide for a reliably fixated implant in particular situations, there is often a tradeoff in attaining an optimal accommodation to the movements of the heart. This means that the implant to some extent impedes those natural movements, and/or possibly interacts with the surrounding anatomy at the implanted site in a detrimental fashion over longer time periods. An annuloplasty implant is intended to function for years and years, so it is critical with long term stability in this regard. A further problem of prior art devices follows from the aforementioned tendency to interfere with the anatomy, leading to reduced tolerances in accommodating for variations in such anatomies of the heart valve. Thus, in addition to being associated with the mentioned problems, existing implants can have a limited degree of adaptability to varying anatomies. This in turn can require a more careful tailoring of the implant and/or procedure to the specific situation at hand, which increases the complexity and the time needed for the overall procedure. This entails a higher risk to the patient and it is thus a further problem of prior art devices.

The above problems may have dire consequences for the patient and the health care system. Patient risk is increased. EP2415421, WO2007/030063 and US2017/007402 disclose an annuloplasty implant with a coiled configuration.

Hence, an improved annuloplasty implant would be advantageous and in particular allowing for avoiding more of the above mentioned problems and compromises, and in particular allowing for improved accommodation to the valve anatomy for secure fixation, during the implantation phase, and for long-term functioning.

### Summary of the Invention

The invention is directed to an annuloplasty implant according to independent claim 1.

Further examples of the invention are defined in the dependent claims

Some examples of the disclosure provide for facilitated fixation of an annuloplasty implant to a target site.

Some examples of the disclosure provide for a more reliable fixation of an annuloplasty to a target site.

Some examples of the disclosure provide for a less time-consuming fixation of an annuloplasty to a target site.

Some examples of the disclosure provide for securing long-term functioning and position of an annuloplasty implant.

Some examples of the disclosure provide for a less complex fixation procedure of an annuloplasty implant.

Some examples of the disclosure provide for a reduced risk of damaging the anatomy of the heart such as the annulus or the valve leaflets.

Some examples of the disclosure facilitate placing fixation units such as sutures at the correct position.

Some examples of the disclosure provide for improved accommodation of an annuloplasty implant to varying anatomies.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which examples of the invention are capable of will be apparent and elucidated from the following description of embodiments of the present invention, reference being made to the accompanying drawings, in which
Fig. 1 is a schematic illustration, in a perspective view, of an annuloplasty implant according to one example;
Figs. 2a-b are schematic illustrations, in top-down views, of an annuloplasty implant according to examples of the disclosure;
Fig. 2c is a schematic illustration, in a perspective view, of an annuloplasty implant according to examples of the disclosure;
Fig. 3a is a schematic illustration, in a top-down view, of an annuloplasty implant according to one example;
Fig. 3b is a schematic illustration, in a side view, of an annuloplasty implant according to one example;
Fig. 4a is a schematic illustration, in a top-down view, of an annuloplasty implant according to one example;
Fig. 4b is a schematic illustration, in a side view, of an annuloplasty implant according to one example;
Fig. 5a is a schematic illustration, in a side view, of an annuloplasty implant according to one example;
Fig. 5b is a schematic illustration, in a side view, of a detail section (A) of the annuloplasty implant in Fig. 5a, according to one example;
Fig. 6a is a schematic illustration, in a side view, of an annuloplasty implant according to one example;
Fig. 6b is a schematic illustration, in a side view, rotated 90 degrees relative the side view of Fig. 6a, of an annuloplasty implant according to one example;
Fig. 7 is a schematic illustration, in a perspective view, of an annuloplasty implant according to one example;
Fig. 8 is a schematic illustration of a cross-section detail, in a side view, of an annuloplasty implant according to one example; and
Fig. 9a is a flow-chart of a method of implanting an annuloplasty implant at a target site in the heart not covered by the invention;
Fig. 9b is a flow-chart of a method of implanting an annuloplasty implant at a target site in the heart not covered by the invention;

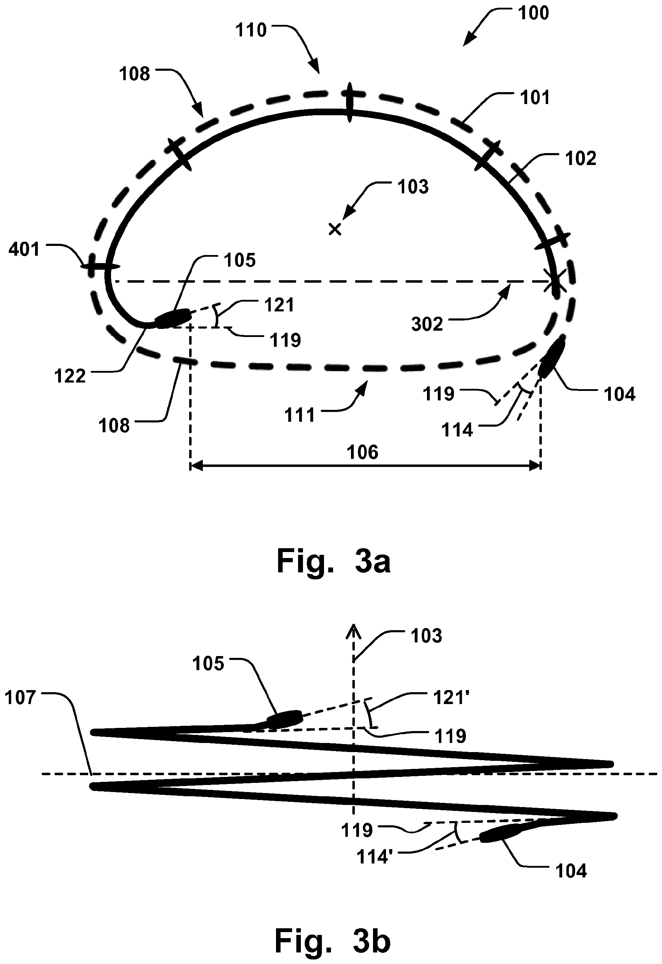

The annuloplasty implant according to the invention is illustrated on fig. 3a, 3b.

### Detailed description

The following description focuses on an embodiment of the present invention applicable to cardiac valve implants such as annuloplasty rings. However, it will be appreciated that the invention is not limited to this application but may be applied to many other annuloplasty implants and cardiac valve implants including for example replacement valves, and other medical implantable devices.

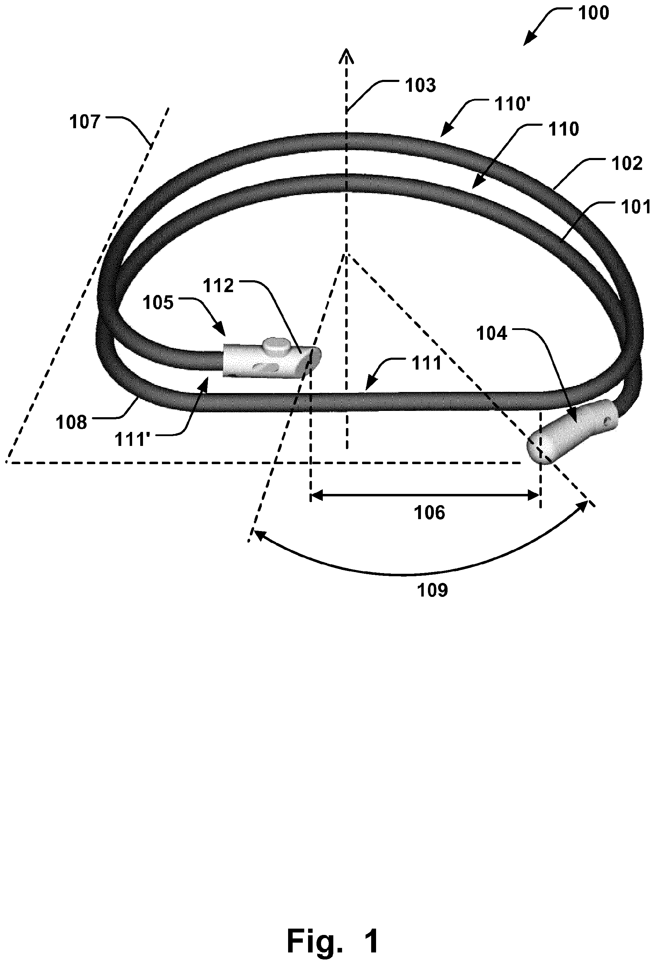

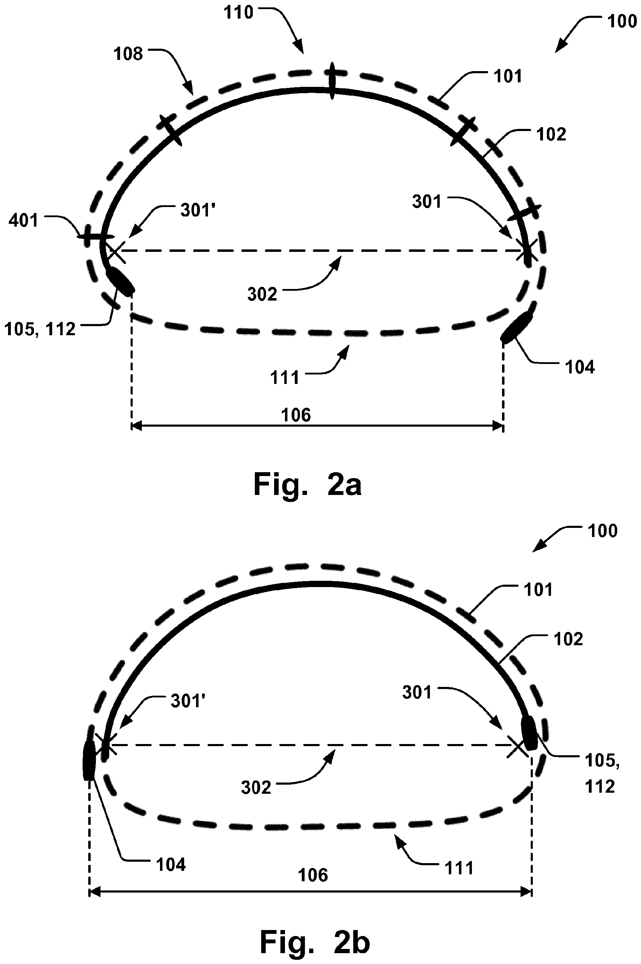

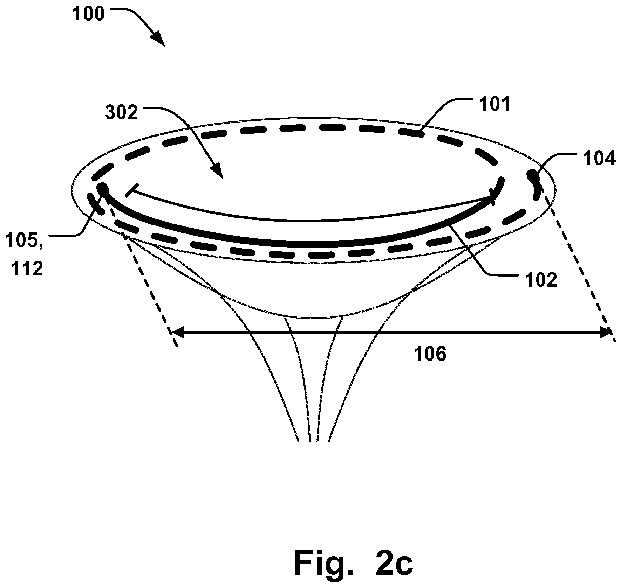

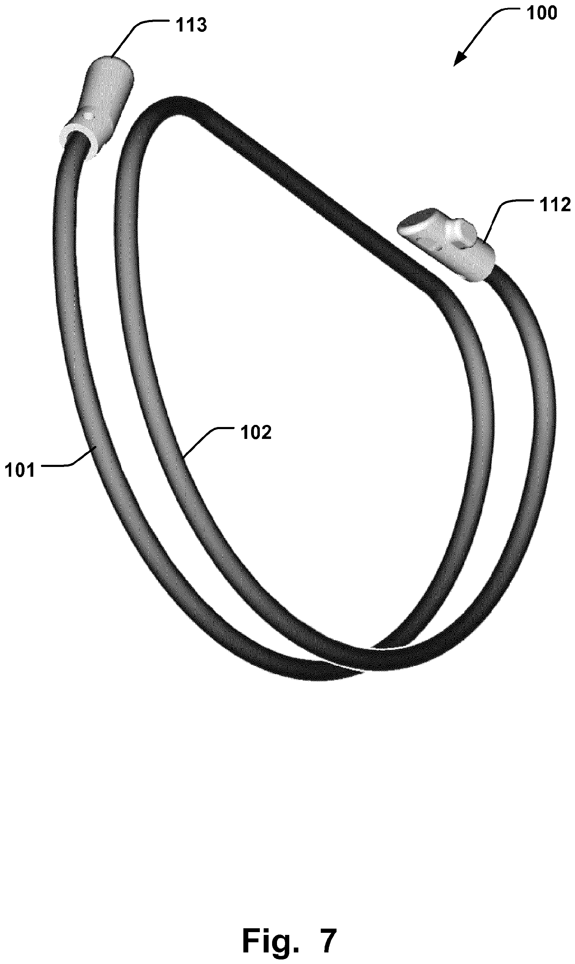

Fig. 1 schematically illustrates an example of an annuloplasty implant 100 comprising a first support member 101 and second support member 102 which are adapted to be arranged as a coil, i.e. in a helix-shape, in a coiled configuration around a central axis 103, as illustrated in Fig. 1. The implant 100 is arranged in the coiled configuration at least when in a relaxed state of the material from which the implant 100 is formed, i.e. free from outside forces acting upon the implant 100. The coil-shaped implant 100 has two free ends 104, 105. The first and second support members 101, 102, and the respective free ends 104, 105, are configured to be arranged on opposite sides of native heart valve leaflets of a heart valve 302. Figs. 2a-b show top-down views of the implant 100 arranged on such opposite sides of a valve 302. The first support member 101 is illustrated with a dashed line and is in these examples arranged on the ventricular side of the heart valve 302, whereas the second support member 202 is arranged on the atrial side of the heart valve 302, as describe in further detail below. Figs. 2a-b show the coil-shaped implant 100 extending between the atrial and ventricular side through any of the two commissures 301, 301'. Fig. 2c is a perspective view corresponding to the configuration shown in Fig. 2b. The first and second support members 101, 102, thus assumes the coiled configuration also when in an implanted state. As explained further below, the implant 100 may comprise a shape-memory material, so that the implant 100 re-assumes the coiled configuration after having been delivered from a catheter to the target site, after having been temporarily restrained in an elongated configuration of the catheter. Alternatively, the implant 100 may maintain a coiled configuration while being delivered to the target site, in which case it may be implanted at the target site for example by incision between the ribs or by opening the chest. The present disclosure, and the associated advantages described for the various examples, applies to both such variants of the implant 100. In the coiled configuration of the implant 100, the two free ends 104, 105, are displaced from each other with a peripheral off-set distance 106 extending in a coil plane 107, as illustrated in Fig. 1. The coil plane 107 is substantially parallel to an annular periphery 108 of the coil and perpendicular to the central axis 103. The central axis 103 is thus perpendicular to the coil plane 107. The coil plane 107 accordingly corresponds to the plane spanned by the annular periphery 108 of the implant 100 in the coiled configuration. The peripheral off-set distance 106 between the two free ends 104, 105, being schematically illustrated in Figs. 1, 2a-c, 3a, 4b, thus extends substantially perpendicular to the central axis 103. This means also that, when the implant 100 is positioned in the implanted state, around the annulus of the heart valve, the two free ends will be separated along the plane of the valve, as illustrated in e.g. Figs. 2a-c. By having such off-set 106 in the plane of the valve 302, the resulting reduced length of the first or second support member 101, 102, will allow for reducing the number of sutures, or other fastening units, required to securely fixate the implant 100 at the valve 302, while at the same time providing for a sufficient overlap of the first and second support member 101, 102, on the opposites sides of the valve to attain a sufficiently strong pinching effect therebetween to fixate the annulus in a modified shape. The fastening units may be integrated with the first and/or second support members 101, 102. In situations, placing fastening units on the anterior side may be associated with high risk. This can therefore be avoided, by having the off-set 106 as specified. Furthermore, the interference of the implant 100 with the movements of the valve 302 will be minimized. Fig. 7 shows a perspective view of the implant 100. Figs. 2a-c, show examples where the peripheral off-set distance 106 between the two free ends 104, 105, have provided a shortened length of the second support member 102, which is positioned at an atrial side of the heart valve 302. Fastening units 401 are illustrated in Fig. 2a on the remaining part of the second support member 102, corresponding to the distance from the free end 105 to the position of the anterior commissure 301, through which the coiled ring 100 extends to the ventricular side of the valve. In Fig. 2b, the off-set distance 106 has been increase even further, compared to Fig. 2a, corresponding substantially to the width of the implant 100 measured in a direction parallel with an anterior side 111 of the implant, which is configured to be placed on an anterior aspect of the heart valve 302. This may also correspond to the widest width of the implant 100, as seen in the examples of e.g. Figs. 2a-c. In Fig. 2b, the first and second support members 101, 102, connect through the posterior commissure 301'. The coiled configuration of the implant seen in Figs. 2a-c may also correspond to the relaxed shape of the material from which the implant 100 is formed, i.e. free from outside forces acting upon the implant 100, eventhough illustrated in the implanted state. The implant 100 may assume such relaxed shape when implanted 100, at least with respect to forces acting in the coil plane 107. The implant 100 may however pinch the tissue of the valve 302, between the first and second support members 101, 102, i.e. with forces acting parallel with the central axis 103.

As mentioned, in addition to the benefits of having fewer fixation points, the separation of the free ends 105, 104, in the plane of the valve 105 reduces the interference with the movement of the valve 302, where otherwise a length of the second support member 102 extending the indicated distance 106 has been observed to repeatedly strike against the valve tissue with the beats of the heart. Such repeated interference may in the long-term lead to tissue damage. Although care could be taken to fixate the implant 100 around its full length in the implantation stage, including the aforementioned interfering portion, if not having the peripheral off-set 106 to separate the free ends 104, 105, there will be an uncertainty in the long-term functioning, and the procedure becomes more complex and time-consuming due to the added requirements. Having the peripheral off-set distance 106 may also further optimize compliance with variations in the valve anatomy, since there will be a minimum of interference with the anatomy. For example, a greater variation the geometry and dynamics of the valve may be accommodated without interfering with the implant 100. This may also provide for increasing the range of valve dimensions which are compliant with an implant 100 of a particular size. Thus, during a particular implantation procedure, the implant 100 provides for better adaptability and the tolerances for variations are increased. Although the anatomy is investigated with various imaging procedures, the complexity of the actual valve and the dynamics of the movement thereof will always pose a significant factor when the implant 100 is being positioned and fixated at the target site.

The off-set distance 106 may correspond to a determined circle sector 109 of the annular periphery 108 by which the two free ends 104, 105, are separated, as illustrated in Fig. 1. The circle sector 109 is given relative the central axis 103. The central axis is positioned at the approximate center point of the coil. The length of the circle sector 109 and the associated distance by which the two free ends 104, 105, are separated may be varied to accommodate various applications and procedures, and may be tailored to various anatomies. It is thus possible to provide a highly compliant implant 100 with a minimum of interference with the natural movements of the heart, and which can be secured more easily.

The annuloplasty implant 100 may comprise at least one posterior bow 110, 110', adapted to conform to a posterior aspect of the heart valve 302, and at least one anterior side 111, 111', adapted to conform to an anterior aspect of the heart valve 302, as illustrated in e.g. Fig. 1. Said determined circle sector 109 may overlap with the at least one anterior side 111, in the aforementioned relaxed state and when in the implanted state. Thus, the two free ends 104, 105, will be separated along the anterior side 111 of the implant 100. It can be particularly beneficial to have the determined circle sector 109 or the off-set distance 106 at the anterior side with respect to achieving the above mentioned advantages, i.e. to avoid having the risk of detrimental damage at the anterior portion of the valve resulting from repeated beating of the implant 100 against the tissue along this portion. And it allows for fixating the implant 100 along the posterior bow 110, 110', only. In the example illustrated in Fig. 1, which will be explained further below, the circle sector 109 overlaps with the anterior side so that the separation of the two free ends 104, 105, provides or results in a first support member 101 with a first anterior side 111 at the ventricular side of the valve 302, and a second support member 102 at the atrial side of the valve 302 that has a second anterior side 111' with a reduced length where the determined circle sector 109 overlaps. As discussed above with respect to Fig. 2b, the off-set distance 106 may be increased to correspond to the width of the implant along the anterior side 111. In this case, the implant 100 may only have a first anterior side 111, as part of the first support member 101, according to Fig. 2b. This may provide for a particularly advantageous positioning and fixation of the implant 100 at the heart valve 302.

Thus, the first support member 101 may be adapted to be arranged on a ventricular side of said heart valve, and the second support member 102 may be adapted to be arranged on an atrial side of said heart valve. The first support member 101 may be adapted to assume an annular ring-shape of substantially 360 degrees on the ventricular side, when in the implanted state, and the second support member 102 may be adapted to assume an annular ring-shape of 360 degrees less the peripheral off-set distance 106 on the atrial side, when in the implanted state. Fastening of the implant 100 on the atrial side can thus be accomplished by fixation of the posterior bow 110', and there will be no interference on the atrial side with the movement of the valve, due to the off-set distance 106 reducing the circle sector of the second support member 102.

The first support member 101 may thus comprise a first posterior bow 110 and a first anterior side 111, and the second support member 102 may comprise a second posterior bow 110', where the second anterior side 111' of the second support member has a length reduced by the off-set distance 106. The length of the second support member 102 may adjusted so that the posterior bow 110 thereof may have a curved transition into an anterior side 111' of a reduced length, as illustrated in e.g. Fig. 1. This may facilitate the interaction of the implant 100 with a delivery device (not shown), e.g. in order to position a connector 112 of the implant 100, described further below, in the correct position relative such delivery device during the implantation procedure. Further, it may enhance and provide for a sufficient strong pinching force between the first and second support members 104, 105, while providing for the above mentioned advantages associated with the off-set 106 between the two free ends 104, 105.

The length of the off-set distance 106 may be between 50-100% of the length of the anterior side 111 of the implant 100, e.g. 100% as mentioned above. The full length of the anterior side 111 may correspond substantially to the portion of the implant 100 that assumes a substantially straight extension, compared to the posterior bow 110, 110', or at least to the portion of the implant 100 that extends between the anterior and the posterior commissures, adjacent the posterior side of the valve 302, i.e. opposite the posterior side where the posterior bow extends between the aforementioned commissures. Fig. 2a schematically illustrates the implant 100 positioned at a valve 302 with an anterior commissure 301, and a posterior commissure 301'. The anterior side 111 extends on the ventricular side between the aforementioned commissures. The length of the off-set distance 106, defining the separation of the two free ends 105, 106, on the ventricular side may thus be varied from assuming the full length of the anterior side between the commissures, as seen in Fig. 2b, to extending to half the distance of the anterior side. This range may provide for an appropriate tradeoff with respect to obtaining the mentioned benefits for facilitating the implantation and improving long-term effects, while still allowing for a stable and secure fixation of the annulus in a modified shape where the valve leaflets can coapt as intended.

The implant 100 may comprise a shape memory material, such as NiTiNol, or another suitable biocompatible alloy that can be heat-set in defined shapes, in a heat treatment procedure. Further, the shape memory material can be conceived as any material that is able to change shape as desired, in response to outside interaction, for example with an energy source, such as providing heat and/or electromagnetic energy, that can be transferred to the implant to change its shape. It is also conceivable that the shape of the implant can be affected by direct mechanical manipulation of the curvature of the ring-shape of the implant 100, e.g. by transferring a force or torque to the implant via a delivery device. Via the various mentioned shape-affecting procedures the implant 100 may thus assume an elongated delivery configuration for advancement in a catheter (not shown), and an implanted shape in the implanted state, assuming a predefined configuration of the shape memory material for positioning at an annulus of the heart valve 302.

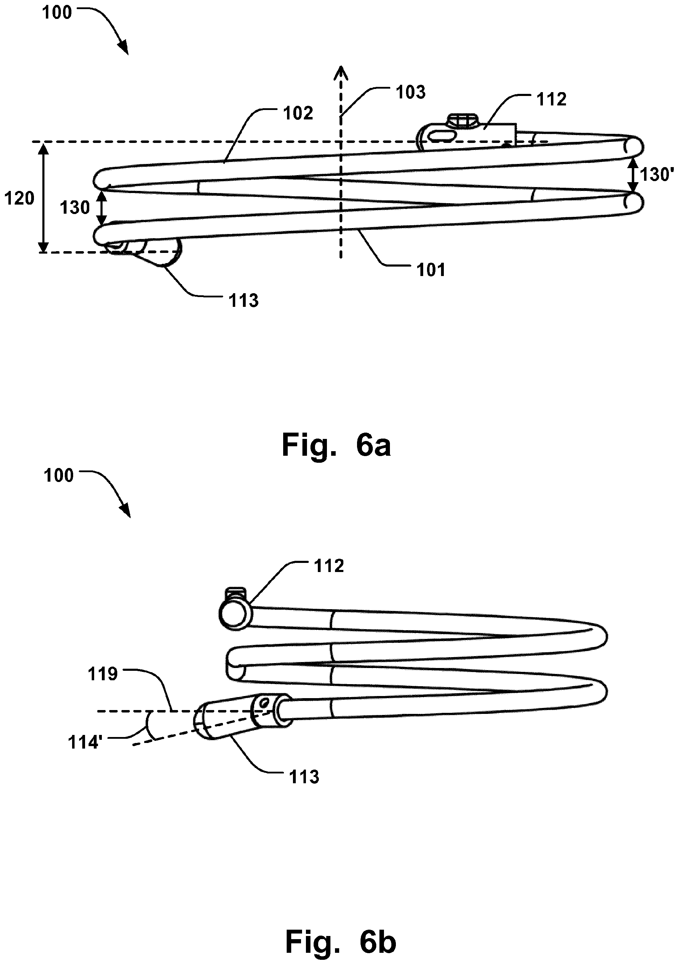

The off-set distance 106 may extend perpendicular to an axial off-set 120 between the two free ends 104, 105. Such axial off-set 120 is illustrated in Fig. 6a. The axial off-set 120 is thus extending substantially parallel to the direction of the central axis 103, i.e. extending as the normal to the coil plane 107. Such axial off-set 120 provide for the advantageous coil- or helix-shaped implant 100 that is easily insertable through the valve 302 to position the first and second support members 101, 102, and the associated two free ends 104, 105, on the opposite sides of the valve 302. Having both the peripheral off-set distance 106 as discussed above and the axial off-set 120 thus provides for an advantageous annuloplasty implant 100 that benefits from the advantages of the helix-shape as well as the improvements discussed with respect to the examples of this disclosure. The first and second support members 101, 102, are separated by a pitch distance 120, 130', as illustrated in Fig. 6a. The pitch distance may be varied for optimization to different anatomies, so that the valve tissue is pinched between the first and second support members 101, 102, effectively. Although Fig. 6a illustrates pitch distances 130 and 130' as being substantially equal, it is also possible that e.g. pitch distance 130 is larger than pitch distance 130'. This may provide for facilitated positioning of the implant 100 at the opposite sides of the heart valve.

The first and second support members 104, 105, may thus be configured to form respective first and second ring-shapes on the opposite sides of the native heart valve leaflets, pinching the leaflets therebetween.

The annuloplasty implant 100 may comprise a connector 112 attached to at least one of the two free ends 104, 105. In the example illustrated in Fig. 1, the connector is arranged on the free end 105 of the second support ring 101. The connector may be adapted to releasably connect to a delivery device (not shown). The connector 112 may this provide for a secure fixation to the delivery device while being delivered to the target site and while the implant 100 is being positioned, e.g. by rotation, into position at the valve 302, and then disconnected from the delivery device to release the implant 100 from therefrom.

The connector 112 may extend substantially parallel with an anterior side 111, 111', of the first or second support member 101, 102, as illustrated in e.g. Fig. 1. This may provide for an advantageous profile of the implant 100 when in the implanted state, as the connector 112 would be aligned with the extension the annulus, as the first and second support members 104, 105. The connector 112 may also extend substantially perpendicular to the anterior side 11 as illustrated in Fig. 2b. The off-set distance 106 may also extend in the coil plane 107 so that the connector 112, and the associated free end 105, are arranged substantially outside of the anterior side 111, as illustrated in the example of Fig. 2a. I.e. the connector 112, and the associated free end 105, are positioned adjacent the commissure 301'. The connector 112, and the associated free end 105, may also be positioned adjacent the commissure 301, as illustrated in Fig. 2b. Having such extended off-set 106 may be particularly advantageous in some applications where it is desired to further minimize the risk of having the free end 105 moving in relation to the anatomy of the beating heart. Fastening unit 401 may be arranged close to the commissure 301', and accordingly close to the connector 112, and the associated free end 105, to minimize the risk of such movement and further provide for the advantageous effects described above.

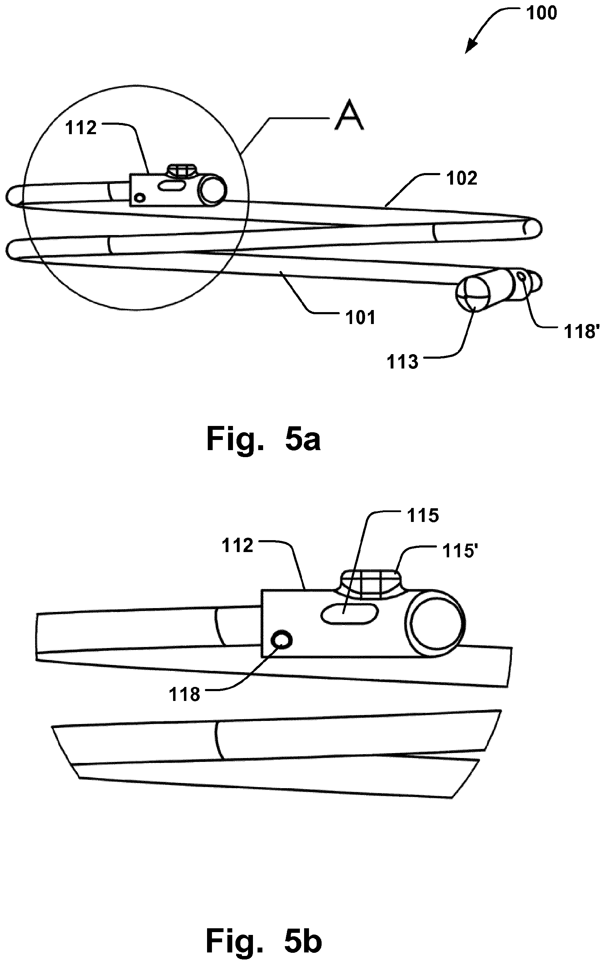

The connector 112 may comprise a recess 115 and/or a protrusion 115' being configured to interlock with a delivery device. Fig. 5a illustrates the implant 100 in a side view, and Fig. 5b is a magnified view of the section "A" in Fig. 5a. In the example of Fig. 5b, the connector 112 comprises a recess 115. The recess 115 may be configured to removably receive a locking member (not shown) that is provided to extend through the recess 115 and interlock with a further recess fixated in relation to the delivery device, such that the locking member fixates the connector 112 in relation to the delivery device. Fig. 5b further illustrates a protrusion 115' on the connector 112, which may be arranged against a corresponding locking surface of the delivery device to provide additional support, and further stabilize the position of the implant 100 in relation to the delivery device.

As further illustrated in Fig. 5b, the connector 112 may be fixated to the implant 100 by a locking pin 118 arranged through a recess of the connector 112 and the implant 100. This provides for a reliable fixation of the connector 112 to the implant 100, and minimizes any risk of dislodgement between the two. The connector 112 is attached to an angled connector extension 122 of the first or second support member 101, 102. Fig. 3a illustrates an example where the second support member 102 is provided with the angled connector extension 122, which extends in a direction radially inwards from the annular periphery 108 of the coil-shaped implant 100. This may be advantageous in the situation the delivery device provides for rotation of the implant 100 around the central axis 103, i.e. around the center 103 of the coil-shaped implant 100 in Fig. 3a, when the implant 100 is about to be rotationally inserted into the valve 302. I.e. by rotating the implant 100 around the central axis 103, there will be a minimum of translator movement in the radial direction, i.e. perpendicular to the center axis, which facilitates the insertion procedure. The connector 112 will in this situation be arranged in a direction which is angled more towards the rotational axis 103, and may provide for a facilitated connection to such delivery device, which will have its longitudinal catheter axis substantially at the center axis 103. Providing for such facilitated connection, with the connector 112 aligned in this radial direction, may allow for a more compact delivery device that is more easily navigated to the target site.

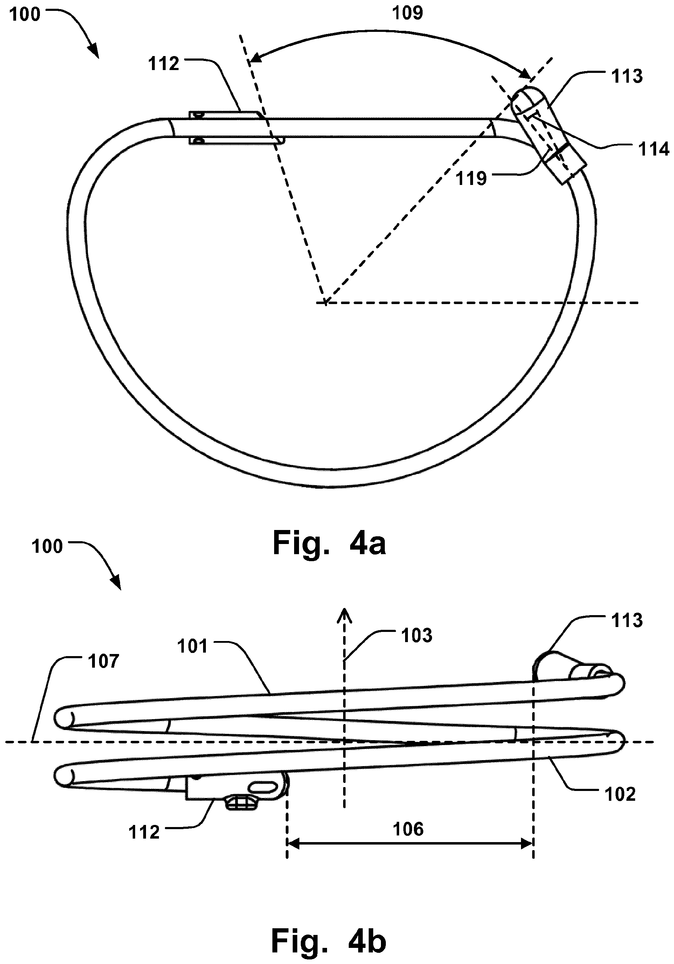

At least one of the two free ends 104, 105, may be arranged at an angle 114, 114', 121, 121', relative a longitudinal extension 119 of the first or second support member 101, 102. Starting with the free end 105 of the second support member, on which a connector 112 is provided, the advantage of having an angle 121 towards the central axis 103 has been described in the previous paragraph. The free end 104 of the first support member 101 may also be provided with an angle 114 in the coil plane 107, relative the longitudinal direction 119 of the support, as illustrated in e.g. Fig. 3a and 4a. This may provide for facilitating the insertion of the free end 104 into the anterior commissure 301 (Fig. 3a) when positioning the implant 100 into position in the valve 302 while not entangling with chordae attached to the leaflet. The angle 114 provides for deflecting the chordae against the free end 104 while positioning the implant 100. At the same time, the angle 114 is chosen so that the free end 104 does not interfere with the adjacent septum of the heart, which may the case when angled too far in the radially outward direction, as illustrated in Figs. 3a and 4a. Turning to Fig. 4a, an outward angle 114 of, for example, around 10 degrees from the longitudinal direction 119 may be a suitable to achieve the advantages described while avoiding interference with the surrounding tissue. The free end 104 may be provided with a blunt tip 113, in order to be atraumatic and minimize the risk of injuring the surrounding tissue. The blunt tip 113 may be fixated to the free end 104 of the implant 100 by a locking pin 118' arranged through a recess of the blunt tip 113 and the implant 100. This provides for a reliable fixation of the blunt tip 113 to the implant 100, and minimizes any risk of dislodgement between the two.

The first and/or second free 104, 105, end may be arranged at an outward angle 114', 121', from the coil plane 107 to extend in a non-parallel direction with the coil plane 107. Turning to the examples of Figs. 3b or 6b, the free end 104 of the first support member 101 may have a downward angle 114', i.e. out of the coil plane 107, to further facilitate insertion of the free end 104 into the anterior commissure 301. The free end 104 will thus more easily catch the commissure when the implant 100 is positioned on the ventricular side of the valve, above the commissure. The free end 105 of the second support member 102 may also have an angle 121' upward, out of the coil plane 107, as illustrated in Fig. 3b. This is particularly advantageous in the case of having an angled connector extension 122 of the free end 105, extending towards the center axis 103, since the upward tilt of the free end 105 will minimize interference with the movements of the leaflets, which will be increasingly more pronounced when moving towards the center axis 103, i.e. the center of the valve.

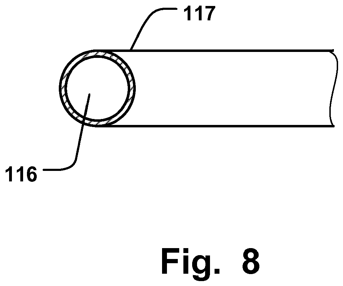

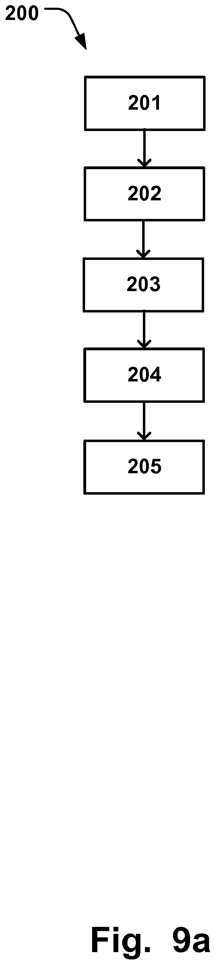

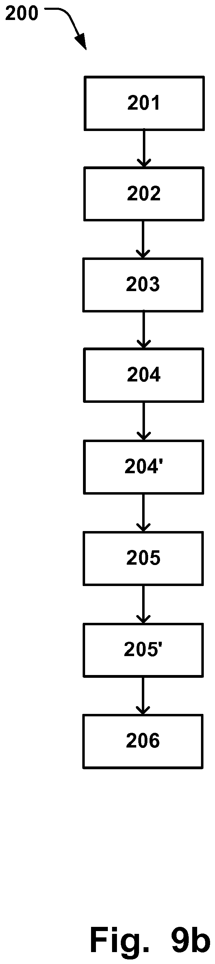

The annuloplasty implant 100 may comprise an inner core 116 of a shape memory material, an outer covering 117 arranged radially outside the inner core material 116 to cover at least part of the inner core 116, as illustrated in Fig. 8. The outer covering 117 is resilient to conform to the inner core 116 during movement of the shape memory material, e.g. when moving from the elongated delivery configuration in inside a delivery catheter, to the relaxed coiled configuration in the implanted state. The outer covering 117 may comprise a first material having surface properties to promote endothelialization. Such material may be a biocompatible alloy such as NiTiNol or it can be stainless steel. The covering 117 may comprise a mesh structure or any structure comprising an irregular, undulated, porous or otherwise non-flat surface to promote endothelialization. The covering may also comprise a polymer, such as Dacron. Simultaneously, the covering 117 has surface properties that provides for a smooth apposition to the surrounding tissue when being implanted, to further ensure safe interaction with the valve as it moves with the beating heart. The methods described hereunder and illustrated on fig. 9a, 9b are not covered by the invention.

A method 200 of implanting an annuloplasty implant 100 at a target site in the heart is exemplified. The method 200 is schematically illustrated in Fig. 9a. The order in which the steps are described should not be construed as limiting, and it is conceivable that the order of the steps may be varied depending on the particular procedure. As discussed, the implant 100 comprises first 101 and second 102 support members. The method 200 comprises delivering 201 the implant from a delivery device, whereupon the first and second support members 101, 102, are arranged as a coil in a coiled configuration around a central axis 103 with two free ends 104, 105. The method 200 further comprise positioning 202 the implant 100 through a first commissure 301, 301', of a valve 302 in the heart; positioning 203 the first and second support members 101, 102, at opposite sides of the valve 302 having a ventricular side and an atrial side. Positioning the first and second support members 101, 102, at opposite sides of the valve 302 comprises positioning 204 the first support member 101 to assume a ring-shape on the ventricular side; and positioning 205 the second support member 102 to assume a ring-shape on the atrial side so that two free ends 104, 105, of the first and second support members 101, 102, are displaced from each other with a peripheral off-set distance 106. As explained above, the peripheral off-set distance 106 is the distance between the two free ends 104, 105, where the peripheral off-set distance 106 extends in a coil plane 107, and where the coil plane 107 is substantially parallel to an annular periphery 108 of the coil and perpendicular to the central axis 103. This provides for the above mentioned advantages ultimately allowing for an improved fixation of the annuloplasty implant 100 and increased patient safety.

A method 200 of implanting an annuloplasty implant 100 at a target site in the heart is schematically illustrated in Fig. 9b. The order in which the steps are described should not be construed as limiting, and it is conceivable that the order of the steps may be varied depending on the particular procedure.

Positioning the first support member 101 to assume a ring-shape on the ventricular side, may comprise positioning 204' the first support member 101 to assume an annular ring-shape of substantially 360 degrees on the ventricular side.

Positioning 205 the second support 102 member may comprise positioning 205' the second support member 102 to assume an annular ring-shape of substantially 360 degrees less the peripheral off-set distance 106 on the atrial side.

The method 200 may comprise fixating 206 the implant 100 by placing fastening units 401 predominantly at a posterior bow 110, 110', of the implant 100. Again, this provides for facilitating the implantation procedure.

## Claims

1. An annuloplasty implant (100) comprising
first (101) and second (102) support members having a coiled configuration in which the first and second support members are arranged as a coil, with two free ends (104, 105), around a central axis (103), wherein the first and second support members and the respective free ends are configured to be arranged on opposite ventricular and atrial sides of native heart valve leaflets of a heart valve,
wherein, in said coiled configuration, said two free ends are displaced from each other with a peripheral off-set distance (106) extending in a coil plane (107), said coil plane being substantially parallel to an annular periphery (108) of said coil and perpendicular to said central axis, the annuloplasty implant further comprising
a connector (112) attached to at least one of said two free ends, and being adapted to releasably connect to a delivery device wherein
said connector is attached to an angled connector extension (122) of said first or second support member, said angled connector extension extending in a direction radially inwards from said annular periphery substantially towards said central axis.

2. Annuloplasty implant according to claim 1, wherein said off-set distance corresponds to a determined circle sector (109) of said annular periphery by which said two free ends are separated.

3. Annuloplasty implant according to claim 2, comprising at least one posterior bow (110, 110') adapted to conform to a posterior aspect of said heart valve, and at least one anterior side (111, 111') adapted to conform to an anterior aspect of said heart valve, and wherein said determined circle sector overlaps with said at least one anterior side.

4. Annuloplasty implant according to any of claims 1 - 3, wherein said first support member is adapted to be arranged on the ventricular side of said heart valve, and said second support member is adapted to be arranged on the atrial side of said heart valve, and wherein said first support member is adapted to assume an annular ring-shape of substantially 360 degrees on said ventricular side, when in said coiled configuration, and wherein said second support member is adapted to assume an annular ring-shape of 360 degrees less said peripheral off-set distance on said atrial side, when in said coiled configuration.

5. Annuloplasty implant according to claim 3 or 4, wherein said first support member comprises a first posterior bow (110) and a first anterior side (111), said second support member comprises a second posterior bow (110'), and wherein a second anterior side (111') of said second support member has a length reduced by said off-set distance.

6. Annuloplasty implant according to any of claims 3 - 5, wherein the length of said off-set distance is between 50-100% of the length of said anterior side.

7. Annuloplasty implant according to any of claims 1 - 6, wherein said implant comprises a shape memory material and has an elongated delivery configuration for advancement in a catheter, and an implanted shape when in an implanted state, assuming a predefined configuration of said shape memory material for positioning at an annulus of said heart valve, and/or
wherein said annuloplasty implant comprises an inner core (116) of a shape memory material and an outer covering (117) arranged radially outside said inner core material to cover at least part of said inner core, wherein said outer covering is resilient to conform to said inner core during movement of said shape memory material, wherein said outer covering comprises a first material having surface properties to promote endothelialization.

8. Annuloplasty implant according to any of claims 1 - 7, wherein said peripheral off-set distance extends perpendicular to an axial off-set (120) between said two free ends, said axial off-set extending substantially parallel to the direction of said central axis.

9. Annuloplasty implant according to any of claims 1 - 8, wherein said first and second support members are configured to form respective first and second ring-shapes on said opposite sides of the native heart valve leaflets to pinch said leaflets therebetween when in said coiled configuration.

10. Annuloplasty implant according to claim 1, wherein said connector extends substantially parallel with an anterior side (111) of said first or second support member.

11. Annuloplasty implant according to claim 1 or 10, wherein said connector comprises a recess (115) and/or a protrusion (115') being configured to interlock with said delivery device, and/or
wherein said connector is fixated to said implant by a locking pin (118) arranged through a recess of said connector and said implant.

12. Annuloplasty implant according to any of claims 1 - 11, wherein at least one of said two free ends is arranged at an angle (114, 114', 121, 121') relative a longitudinal extension (119) of said first or second support member.

13. Annuloplasty implant according to claim 12, wherein said first and/or second free end is arranged at an outward angle (114', 121') from said coil plane to extend in a non-parallel direction with said coil plane.

## Patentansprüche

1. Annuloplastie-Implantat (100), umfassend
erste (101) und zweite (102) Stützelemente mit einer aufgerollten Konfiguration, in der die ersten und zweiten Stützelemente als eine Spirale mit zwei freien Enden (104, 105) um eine Mittelachse (103) angeordnet sind, wobei die ersten und zweiten Stützelemente und die jeweiligen freien Enden dazu ausgelegt sind, auf gegenüberliegenden Ventrikel- und Vorhofseiten nativer Herzklappensegel einer Herzklappe angeordnet zu werden,
wobei die zwei freien Enden in der aufgerollten Konfiguration mit einem peripheren Versatzabstand (106), der sich in einer Spiralebene (107) erstreckt, voneinander versetzt sind, wobei die Spiralebene im Wesentlichen parallel zu einem ringförmigen Umfang (108) der Spirale und senkrecht zur Mittelachse ist, wobei das Annuloplastie-Implantat ferner
ein Verbindungsglied (112) umfasst, das an mindestens einem der zwei freien Enden befestigt ist und das zur lösbaren Verbindung mit einer Freisetzungsvorrichtung angepasst ist, wobei
das Verbindungsglied an einer angewinkelten Verbindungsgliedverlängerung (122) des ersten oder zweiten Stützelements befestigt ist, wobei sich die angewinkelte Verbindungsgliedverlängerung in einer Richtung radial einwärts vom ringförmigen Umfang im Wesentlichen zur Mittelachse erstreckt.

2. Annuloplastie-Implantat nach Anspruch 1, wobei der Versatzabstand einem ermittelten Kreissektor (109) des ringförmigen Umfangs, um den die zwei freien Enden voneinander getrennt sind, entspricht.

3. Annuloplastie-Implantat nach Anspruch 2, umfassend mindestens einen posterioren Bogen (110, 110'), der dazu angepasst ist, sich an einen posterioren Aspekt der Herzklappe anzuschmiegen, und mindestens eine anteriore Seite (111, 111'), die dazu angepasst ist, sich an einen anterioren Aspekt der Herzklappe anzuschmiegen, und wobei der ermittelte Kreissektor die mindestens eine anteriore Seite überlappt.

4. Annuloplastie-Implantat nach einem der Ansprüche 1-3, wobei das erste Stützelement dazu angepasst ist, auf der Ventrikelseite der Herzklappe angeordnet zu werden, und das zweite Stützelement dazu angepasst ist, auf der Vorhofseite der Herzklappe angeordnet zu werden, und wobei das erste Stützelement dazu angepasst ist, eine Kreisringgestalt von im Wesentlichen 360 Grad auf der Ventrikelseite in der aufgerollten Konfiguration anzunehmen, und wobei das zweite Stützelement dazu angepasst ist, eine Kreisringgestalt von 360 Grad abzüglich des peripheren Versatzabstands auf der Vorhofseite in der aufgerollten Konfiguration anzunehmen.

5. Annuloplastie-Implantat nach Anspruch 3 oder 4, wobei das erste Stützelement einen ersten posterioren Bogen (110) und eine erste anteriore Seite (111) umfasst, wobei das zweite Stützelement einen zweiten posterioren Bogen (110') umfasst und wobei eine zweite anteriore Seite (111') des zweiten Stützelements eine Länge aufweist, die um den Versatzabstand reduziert ist.

6. Annuloplastie-Implantat nach einem der Ansprüche 3-5, wobei die Länge des Versatzabstands zwischen 50-100% der Länge der anterioren Seite beträgt.

7. Annuloplastie-Implantat nach einem der Ansprüche 1-6, wobei das Implantat ein Formgedächtnismaterial umfasst und eine längliche Freisetzungskonfiguration zum Vorschieben in einem Katheter und eine implantierte Gestalt in einem implantierten Zustand aufweist, die eine vorgegebene Konfiguration des Formgedächtnismaterials zur Platzierung an einem Annulus der Herzklappe annimmt, und/oder
wobei das Annuloplastie-Implantat einen inneren Kern (116) aus einem Formgedächtnismaterial und eine äußere Abdeckung (117), die radial außerhalb des inneren Kernmaterials angeordnet ist, um zumindest einen Teil des inneren Kern abzudecken, umfasst, wobei die äußere Abdeckung elastisch ist, um sich während einer Bewegung des Formgedächtnismaterials an den inneren Kern anzuschmiegen, wobei die äußere Abdeckung ein erstes Material mit Oberflächeneigenschaften zur Förderung der Endothelialisierung umfasst.

8. Annuloplastie-Implantat nach einem der Ansprüche 1-7, wobei sich der periphere Versatzabstand senkrecht zu einem axialen Versatz (120) zwischen den zwei freien Enden erstreckt, wobei sich der axiale Versatz im Wesentlichen parallel zur Richtung der Mittelachse erstreckt.

9. Annuloplastie-Implantat nach einem der Ansprüche 1-8, wobei die ersten und zweiten Stützelemente dazu angepasst sind, jeweilige erste und zweite Ringformen auf den gegenüberliegenden Seiten der nativen Herzklappensegel unter Einklemmen der Segel dazwischen in der aufgerollten Konfiguration zu bilden.

10. Annuloplastie-Implantat nach Anspruch 1, wobei sich das Verbindungsglied im Wesentlichen parallel mit einer anterioren Seite (111) des ersten oder zweiten Stützelements erstreckt.

11. Annuloplastie-Implantat nach Anspruch 1 oder 10, wobei das Verbindungsglied eine Aussparung (115) und/oder einen Vorsprung (115') umfasst, die bzw. der zur Verriegelung mit der Freisetzungsvorrichtung ausgelegt ist, und/oder
wobei das Verbindungsglied durch einen Verriegelungsstift (118), der durch eine Aussparung des Verbindungsglieds und das Implantat angeordnet ist, am Implantat fixiert ist.

12. Annuloplastie-Implantat nach einem der Ansprüche 1-11, wobei zumindest eines der zwei freien Enden in einem Winkel (114, 114', 121, 121') bezüglich einer Längsverlängerung (119) des ersten oder zweiten Stützelements angeordnet ist.

13. Annuloplastie-Implantat nach Anspruch 12, wobei das erste und/oder zweite freie Ende in einem Auswärtswinkel (114', 121') von der Spiralebene angeordnet ist, um sich in einer nicht-parallelen Richtung mit der Spiralebene zu erstrecken.

## Revendications

1. Implant d'annuloplastie (100) comprenant
des premier (101) et second (102) éléments de support possédant une configuration enroulée dans laquelle les premier et second éléments de support sont agencés sous la forme d'un enroulement, avec deux extrémités libres (104, 105), autour d'un axe central (103), dans lequel les premier et second éléments de support et les extrémités libres respectives sont conçus pour être agencés sur les côtés ventriculaire et auriculaire opposés de feuillets de valvule cardiaque native,
dans lequel, dans ladite configuration enroulée, lesdites deux extrémités libres sont déplacées l'une par rapport à l'autre avec une distance de décalage périphérique (106) s'étendant dans un plan d'enroulement (107), ledit plan d'enroulement étant sensiblement parallèle à une périphérie annulaire (108) dudit enroulement et perpendiculaire audit axe central, l'implant d'annuloplastie comprenant en outre
un raccord (112) fixé à au moins une desdites deux extrémités libres, et étant conçu pour se raccorder de manière libérable à un dispositif de pose en place dans lequel
ledit raccord est fixé à une extension inclinée (122) de raccord dudit premier ou second élément de support, ladite extension inclinée de raccord s'étendant dans une direction radialement vers l'intérieur depuis ladite périphérie annulaire sensiblement vers ledit axe central

2. Implant d'annuloplastie selon la revendication 1, dans lequel ladite distance de décalage correspond à un secteur circulaire déterminé (109) de ladite périphérie annulaire par lequel sont séparées lesdites deux extrémités libres.

3. Implant d'annuloplastie selon la revendication 2, comprenant au moins une cambrure postérieure (110, 110') conçue pour épouser la forme d'un aspect postérieur de ladite valvule cardiaque, et au moins un côté antérieur (111, 111') conçu pour épouser la forme d'un aspect antérieur de ladite valvule cardiaque, et dans lequel ledit secteur circulaire déterminé chevauche ledit au moins un côté antérieur.

4. Implant d'annuloplastie selon l'une quelconque des revendications 1 à 3, dans lequel ledit premier élément de support est conçu pour être agencé sur le côté ventriculaire de ladite valvule cardiaque, et ledit second élément de support est conçu pour être agencé sur le côté auriculaire de ladite valvule cardiaque, et dans lequel ledit premier élément de support est conçu pour adopter une forme d'anneau circulaire de sensiblement 360 degrés sur ledit côté ventriculaire, dans ladite configuration enroulée, et dans lequel ledit second élément de support est conçu pour adopter une forme d'anneau circulaire de 360 degrés moins ladite distance de décalage périphérique sur ledit côté auriculaire, dans ladite configuration enroulée.

5. Implant d'annuloplastie selon la revendication 3 ou 4, dans lequel ledit premier élément de support comprend une première cambrure postérieure (110) et un premier côté antérieur (111), ledit second élément de support comprend une seconde cambrure postérieure (110'), et dans lequel un second côté antérieur (111') dudit second élément de support a une longueur réduite par ladite distance de décalage.

6. Implant d'annuloplastie selon l'une quelconque des revendications 3 à 5, dans lequel la longueur de ladite distance de décalage est entre 50 et 100 % de la longueur dudit côté antérieur.

7. Implant d'annuloplastie selon l'une quelconque des revendications 1 à 6, dans lequel ledit implant comprend un matériau à mémoire de forme et possède une configuration de pose en place allongée pour un mouvement d'avance dans un cathéter, et une forme implantée dans un état implanté, adoptant une configuration prédéfinie dudit matériau à mémoire de forme pour un positionnement au niveau d'un anneau de ladite valvule cardiaque, et/ou
dans lequel ledit implant d'annuloplastie comprend un coeur interne (116) en matériau à mémoire de forme et un recouvrement externe (117) agencé radialement à l'extérieur dudit matériau de coeur interne pour recouvrir au moins une partie dudit coeur interne, dans lequel ledit recouvrement externe est résilient pour épouser la forme dudit coeur interne pendant un mouvement dudit matériau à mémoire de forme, dans lequel ledit recouvrement externe comprend un premier matériau possédant des propriétés de surface pour favoriser l'endothélialisation.

8. Implant d'annuloplastie selon l'une quelconque des revendications 1 à 7, dans lequel ladite distance de décalage périphérique s'étend perpendiculairement à un décalage axial (120) entre lesdites deux extrémités libres, ledit décalage axial s'étendant sensiblement parallèlement à la direction dudit axe central.

9. Implant d'annuloplastie selon l'une quelconque des revendications 1 à 8, dans lequel lesdits premier et second éléments de support sont conçus pour former des première et seconde formes annulaires respectives sur lesdits côtés opposés des feuillets de valvule cardiaque native pour pincer lesdits feuillets entre ceux-ci dans ladite configuration enroulée.

10. Implant d'annuloplastie selon la revendication 1, dans lequel ledit raccord s'étend sensiblement parallèlement à un côté antérieur (111) dudit premier ou second élément de support.

11. Implant d'annuloplastie selon la revendication 1 ou 10, dans lequel ledit raccord comprend un renfoncement (115) et/ou une saillie (115') conçus pour s'interverrouiller avec ledit dispositif de pose en place, et/ou
dans lequel ledit raccord est fixé audit implant par une broche de verrouillage (118) agencée à travers un renfoncement dudit raccord et dudit implant.

12. Implant d'annuloplastie selon l'une quelconque des revendications 1 à 11, dans lequel au moins une desdites deux extrémités libres est agencée selon un angle (114, 114', 121, 121') par rapport à une extension longitudinale (119) dudit premier ou second élément de support.

13. Implant d'annuloplastie selon la revendication 12, dans lequel ladite première et/ou ladite seconde extrémité libre sont agencées selon un angle externe (114', 121') à partir dudit plan d'enroulement pour s'étendre dans une direction non parallèle avec ledit plan d'enroulement.
